# EUROPEAN PATENT SPECIFICATION

(11) **EP 3 071 238 B1**
(45) Date of publication and mention of the grant of the patent: **01.05.2019**
(21) Application number: 14830584.0
(22) Date of filing: 19.11.2014
(51) Int. Cl.: A61P 35/00, A61P 29/00, A61P 9/00, C07D 207/46, C07D 209/12, A61K 47/60, A61K 47/55

(54) **CONJUGATE COMPRISING INDOLE-3-CARBINOL FOR MEDICAL USE**
KONJUGAT MIT INDOL-3-CARBINOL FÜR MEDIZINISCHE ZWECKE
CONJUGUÉ COMPRENANT DE L'INDOLE-3-CARBINOL À USAGE MÉDICAL

(30) Priority: 20.11.2013 IT MI20131929
(43) Date of publication of application: 28.09.2016
(73) Proprietor: Dom Terry International S.r.l., 20121 Milano (IT)
(72) Inventor: RASTRELLI, Luca, 80126 Napoli (IT); TERENZIO, Domenlco, 04022 Fondi, Latina (IT)
(74) Representative: Croce, Valeria
(86) International application number: PCT/IB2014/066168
(87) International publication number: WO 2015/075647

(56) References cited:
- WO-A2-2010/118339
- US-A1- 2011 105 413
- GREENWALD, R. B. ET AL.: "Poly(ethylene glycol) Prodrugs of the CDK Inhibitor, Alsterpaullone (NSC 705701): Synthesis and Pharmacokinetic Studies", BIOCONJUGATE CHEMISTRY, vol. 15, 14 August 2004 (2004-08-14), pages 1076-1082, XP002723837,
- IZET M KAPETANOVIC ET AL: "Pharmacokinetics and enhanced bioavailability of candidate cancer preventative agent, SR13668 in dogs and monkeys", CANCER CHEMOTHERAPY AND PHARMACOLOGY, SPRINGER, BERLIN, DE, vol. 65, no. 6, 16 September 2009 (2009-09-16), pages 1109-1116, XP019800781, ISSN: 1432-0843
- ROBERTS M J ET AL: "Chemistry for peptide and protein PEGylation", ADVANCED DRUG DELIVERY REVIEWS, ELSEVIER, AMSTERDAM, NL, vol. 54, no. 4, 17 June 2002 (2002-06-17), pages 459-476, XP002293146, ISSN: 0169-409X, DOI: 10.1016/S0169-409X(02)00022-4

## Description

The present invention relates to an indole-3-carbinol conjugate for medical use, and, particularly, for the prevention and treatment of neoplastic diseases, inflammatory diseases, cardiovascular diseases and other diseases, particularly those ascribable to the metabolic syndrome.

### Background of the art

The anti-tumoral drugs used today in therapy are an heterogeneous group of compounds inhibiting the growth of a neoplasm, having very different structure and mechanisms of action. However, a common element can be found in the high cytotoxicity, especially on the tissues characterized by a high cell proliferation. The limited success of a pharmacological therapy that uses the traditional compounds is also due to a poor selectivity to the cancer cells, which significantly decreases the therapeutic effect and produces undesired side effect on other tissues. Another problem is represented by the limited half-life of the compounds, due to a rapid kidney clearance and a quick inactivation and enzymatic degradation.

Therefore, it is known the need to develop new medicaments having anti-tumoral activity, which are more selective, less toxic, and which are characterized by better pharmacologic properties.

### Indole-3-carbinol

Indole-3-carbinol (abbreviated with I3C) is a natural-occurring substance contained in the vegetables of the Cruciferae family, especially broccoli, white cabbage, Brussels sprouts and cauliflower. In 1997, it ability in stopping the development of breast cancer has been shown by positively acting on the metabolism of estrogens and stopping, as tamoxifen, the cell cycle.

Furthermore, indole-3-carbinol is capable of adjusting the dependence of many cancers from hormones, since it binds the nuclear estrogen receptor by preventing the cell proliferation induced by the latter ones.

In a number of *in vitro* and *in vivo* studies, it has been possible to show that this compound inhibits up to 90% the development of the tumor cells that are positive to the estrogen receptor. Also in breast carcinomas that are negative to the estrogen receptor, the administration of indole-3-carbinol inhibits the cell growth. In this type of tumors, however, tamoxifen has no efficacy. In other studies, indole-3-carbinol showed to be able to suppress the growth of various tumor cells, including colon tumor cells by stopping the cell cycle in G1/S and inducing apoptosis *in vitro.*

I3C acts as an antagonist of the androgen receptor, and it is capable of inhibiting the proliferation of the tumor cells in the cases of prostate cancer. Furthermore, it is a powerful inducer of the cytochrome P450 and intervenes in the metabolism of estrogens. These seem to intervene also in the pathogenesis of Recurrent Respiratory Papillomatosis.

Clinical studies have been carried out, by the use of neat I3C, as a dietary supplement, with good results for this disease.

In other studies, the anti-inflammatory activity *in vivo* and *in vitro* of I3C has been assessed. Indole-3-carbinol has shown to inhibit the expression and activity of inducible nitric oxide synthetase (iNos) in cells stimulated by LPS, assessing the production of nitrites and the inhibition of the NO release. In fact, the constant production of nitric oxide is connected to the inflammation process and hence to the carcinogenesis process.

I3C reduces the production of other pro-inflammatory mediators, such as IL-6 and IL-1β suggesting that it may provide a therapeutic strategy that is useful in the treatment of several inflammatory diseases.

The I3C treatment results in a body weight loss and a reduction in building-up fat and macrophages infiltrated in the adipose tissue of the epididymis of obese mice fed with a fat-rich regimen. These reductions were associated to a better glucose tolerance.

Furthermore, the platelet anti-aggregating effect of I3C has been tested by assessing the aggregation inhibition induced by ADP, collagen, adrenaline, arachidonic acid on human blood samples. I3C has shown to inhibit in a dose-dependent manner the collagen-induced platelet aggregation. *In vivo,* I3C suppresses the death of rats with pulmonary thrombosis induced by intravenous collagen and adrenaline injection.

### Carrier

The poly-(ethylene glycol) (PEG) is a linear synthetic polymer having a low polidispersity composed of repeats of oxyethylene units.

Due to its biocompatibility and non-toxicity, it finds a wide use in the food, cosmetic, and pharmaceutic fields.

### Folic acid receptor

The receptor for the vitamin folic acid (FR), otherwise known as membrane protein having a high affinity for folate, is a glycoprotein with a molecular weight of about 38 kDa. Three isoforms have been identified in humans, named α, β, γ/ γ'. While a high expression of FR has been frequently noticed in various types of human cancer, the receptor is generally absent in most of normal tissues, except for the choroid plexa, placenta and, to a lower extent, in the lung, thyroid, and kidneys. FR is frequently overexpressed in tumor cells in culture and in epithelial tumors, particularly in the ovarian carcinoma (90% of the cases), of which it is also a useful marker. Other types of tumor overexpress this receptor, particularly the endometrium, brain, lung, breast and kidney cancers. The FR-α isoform is overexpressed in malignant epithelial tumors, while the FR-β isoform in sarcomas and myeloid leukemias, thus allowing targeting a large number of neoplasms. FR-β is further overexpressed in monocytes and activated macrophages, making the targeting very useful also in the inflammatory processes.

Further, it should be pointed out that the receptor is physiologically present only in the apical membrane of the epithelial cells, therefore, this being inaccessible to the blood flow, the cell is protected by the action of medicaments derivatized with folic acid. After the malignant conversion, the cell polarity is lost, and the receptor becomes accessible to the blood flow.

### Summary of the invention

The drawbacks of the known antitumoral medicaments and some limitations of use of the indole-3-carbinol, particularly the low molecular weight, the instability in water and in the acidic environment of the stomach, are overcome by the pharmaceutical compounds of the present invention, which have shown to be particularly efficient in the prevention and treatment of cancer, inflammatory and cardiovascular diseases, and other diseases.

### Object of the invention

In a first object, the present invention therefore discloses new pharmaceutical compounds represented by conjugates of indole-3-carbinol.

According to a further aspect, such compounds are for use in the treatment and prevention of cancer in humans and animals.

In a preferred aspect, the conjugates of the invention are for use for the treatment and the prevention of carcinomas, sarcomas, lymphomas, melanomas, mesotheliomas, leukemias, myelomas and other neoplastic diseases.

Particularly, the conjugates of the invention are for use for the treatment and prevention of breast, colon, prostate, lung, nose, throat, brain and ovary cancer.

In a further aspect, the conjugates of the invention are for use in the treatment of inflammatory diseases, by virtue of the suppression exerted on the activity of the activated macrophages.

In another aspect, the conjugates of the invention, by stabilizing I3C, can find use in the field of both primary and secondary prevention of cardiovascular diseases.

In a further aspect, the conjugates of the invention are for use as antioxidants that are capable of preventing several diseases associated to oxidative stress.

Herein, a process is described, for the preparation of the conjugates of indole-3-carbinol or derivatives thereof.

Furthermore, a pharmaceutical or nutraceutical composition is described, comprising one or more of the conjugates of the invention.

### Detailed description of the invention

According to a first object of the invention, conjugates of indole-3-carbinol, or a derivative thereof are described, of the general formula:

**Bₓ-(S)_{y}-L-(S')_{z}-Dₙ** **(I)**

wherein:
B is represented by folic acid (F);
**x** can be 0 or 1 or 2 and it is preferably 0 or 1;
**S** is preferably represented by lysine (Lys), which may have a function of spacer when L is a homobifunctional carrier, or S is a bifunctional carrier comprising two activable or derivatizable functions, such as L-2-aminoadipic acid (AD);
**y** is 0, or it is 1 when L is a homofunctional carrier, or it is 2;
**L** is a carrier selected from polyethylene glycol (herein below abbreviated with PEG or (PEG)ₙ), or a derivative thereof which can be homobifunctional (for example, HOOC-(PEG)ₙ-COOH) or heterobifunctional (for example, H₂N-(PEG)ₙ-COOH) according to the fact that it carries or not at its end a same reactive group (carboxyl protected with ester or not or amine) optionally suitably activated. The carrier can be a different polymerization degree and it is preferably characterized by a molecular weight ranging between 100-200,000 (g/mol). Preferred examples of PEG are PEG_{20,000}, PEG_{4.800}, PEG₆₀₀, and PEG₅₅₀.
**S'** is a monofunctional or bifunctional spacer comprising one or two activable or derivatizable functions (for example, two carboxy functions), respectively; it is preferably a bifunctional carrier selected from L-2-aminoadipic acid (AD) and β-glutamic acid (β-Glu) ;
**z** is the number of spacers linked to the carrier, and it is 0 (in the case that D is directly linked to the carrier) or 1 or 2;
**D** is the active ingredient selected from indole-3-carbinol (I3C) or a derivative thereof comprising N-acyl derivatives comprising a straight or branched carbonyl chain C₁-C₆ and preferably represented by N-acetyl derivatives; N-alkoxy derivatives comprising a straight or branched C₁-C₆ carbonyl chain and preferably represented by: N-methoxy, N-ethoxy, N-propoxy, N-butoxy; N-aryl or N-heteroaryl derivatives comprising one or more heteroatoms selected from nitrogen, oxygen and sulphur, or N-benzyl, N-alkyl comprising a straight or branched C₁-C₆ carbonyl chain optionally comprising one or more heteroatoms selected from nitrogen, oxygen and sulphur, N-alkenyl or N-alkinyl comprising a straight or branched C₂-C₆ carbonyl chain comprising one or more insaturations and optionally further comprising one or more heteroatoms selected from nitrogen, oxygen and sulphur;
**n** is the number of molecules of D and is 1 or 2 or 3 or 4.

In accordance with a preferred aspect, the conjugate of the present invention is a compound having formula:
**PEG-I3C**
**I3C-PEG-I3C**
**PEG-AD-(I3C)₂**
**PEG-βGLU-(I3C)₂**
**B-Lys-PEG-I3C**
**B-Lys-PEG-AD-(I3C)₂**
**B-Lys-PEG-βGLU-(I3C)₂**
**F-PEG-I3C**
**F-PEG-AD-(I3C)₂**
**(I3C)₂-AD-PEG-AD-(I3C)₂**
**F-PEG-βGLU-(I3C)₂**
**F-Lys-PEG-I3C**
**F-Lys-PEG-AD-(13C)₂**
**F-Lys-PEG-βGLU-(I3C)₂**
**F₂-AD-PEG-I3C**
**F₂-AD-PEG-βGLU-(I3C)₂**
**F₂-AD-PEG-AD-(I3C)₂**
wherein F is folic acid; Lys is lysine; PEG is as defined above, therefore it is polyethylene glycol or a derivative thereof, such as, for example, monomethoxy polyethylene glycol (mPEG), or it is homobifunctional (for example, HOOC-(PEG)ₙ-COOH) or heterobifunctional (for example, H₂N-(PEG)ₙ-COOH) PEG; wherein AD is L-2-aminoadipic acid and β-Glu is β-glutamic acid, and wherein I3C is indole-3-carbinol (I3C) or a derivative thereof as described above. Furthermore, a process for the preparation of the conjugates of the invention is described.

The preparation of such conjugates comprises a first step I) of activation of the carrier, in turn comprising the steps of:
Ia) introducing in the carrier a suitable activable reactive group (gr):

   **L → L-gr;**
Ib) activation of the *carrier* by activating the reactive group:

   **L-gr → L-gr***

In a second step II) the activation of the ligand B is performed:

**B → B*.**

In the third step III) of the preparation of the conjugates of the invention, the activated ligand is conjugated to the carrier:

**B* + L-gr* → B-L-gr***

In the last step IV) there is the conjugation with the active ingredient:

**B-L-gr* + D → B-L-D.**

In a preferred aspect of the method, the activation of the carrier (step Ia) initially comprises the treatment thereof with a suitable base, such as potassium tert-butylate in a suitable solvent, followed by treatment with t-butylbromoacetate so as to obtain the insertion of a reactive group which can be activated, such as, for example, -CH₂COOH, on the carrier.

The step Ia) cannot be performed when a carrier is used, which already comprises a suitable activable reactive group; also or only the step Ib) can be avoided starting from a carrier comprising a suitable activated reactive group.

In the step Ib), the carboxyl group is derivatized, for example by treatment with N-hydroxysuccinimide (NHS) in the presence of N,N'-dicyclohexylcarbodiimide (DCC).

The steps Ia) and Ib) can be repeated in the case the insertion of more reactive groups is desired.

Instead, as regards the activation of the ligand (step II), this is obtained for example through the esterification with N-hydroxysuccinimide (NHS) in the presence of N,N'-dicyclohexylcarbodiimide (DCC).

In a preferred aspect of the method, the step III) of conjugating the ligand to the carrier is obtained by forming an amide bond between the succinimide ester of the ligand and the amine group of the carrier (for example, the carrier can be heterofunctional PEG).

The above-described steps II) and III) cannot be performed in the case that the compound of the invention does not comprise a targeting group, such as, for example, when y and x are 0.

In a preferred aspect of the present method, in the step III), a carrier:ligand molar ratio of 1:4 is used.

The step of preparation IV) of the conjugate of the invention comprises the subsequent formation of the bond, for example, an ester bond, between the activated reactive group of the carrier and the reactive group of the active ingredient (for example, the -OH group of indole-3-carbinol).

Such step can be repeated as a function of the number of reactive groups that are inserted and activated.

According to a preferred aspect of the method, in the step IV) a carrier:active ingredient molar ratio is used, ranging between about 1:1 and 1:5, and preferably of about 1:3.

In the case that the bond between carrier and active ingredient occurs by a spacer S', the process for preparing the conjugates of the invention provides for the use of a heterobifunctional carrier (carrying two reactive groups: gr and gr', which are mutually different) and the process comprises the steps of:
Ia') protecting one of the reactive groups of the carrier:

**gr-L-gr' → #L-gr'**

followed by the step Ib') of activating the free reactive group (gr'):

**#L-gr' → #L-gr'***

Subsequently, a step Ic') of conjugation with a spacer S' is provided for:

**#L-gr'* + S' → #L-S'**

and a step Id') of deprotecting the protected reactive group of the *carrier:*

**#L-S' → gr-L-S'.**

In the successive step Ie'), one proceeds with the conjugation with the ligand by the available gr:

**gr-L-S' + B → B-L-S'**

Subsequently, one proceeds with the step If') of activating the reactive group of the spacer:

**B-L-S' → B-L-S'***

and then with the step Ig') of conjugation with the active ingredient:

**B-L-S'* + D → B-L-S'-D.**

The process described above can be adapted and modified according to the needs in accordance with the techniques known to those skilled in the art.

In a preferred aspect of the invention, the heterobifunctional carrier is represented by H₂N-(PEG)ₙ-COOH, which can be suitably blocked at the amine end with t-butyloxycarbonyl (Boc). As regards the other reactive group (carboxyl), this can be preferably activated with N-hydroxysuccinimide.

In a preferred aspect of the invention, the spacers S' used are selected from (L)-2-aminoadipic acid and β-glutamic acid.

As regards the deprotection of the *carrier,* this is obtained in the case of the protecting group Boc preferably with trifluoroacetic acid.

The activation of the carboxyl groups of the spacers S' are obtained by reaction with N-hydroxysuccinimide in the presence of DCC.

In a preferred aspect of the invention, in the case that the bond between the active ingredient and the carrier is obtained by a spacer S', a carrier:active ingredient molar ratio is used, ranging between about 1:1 and 1:5 and preferably of about 1:3.

In the case that the spacer is a bifunctional carrier, for example, L-2-aminoadipic acid (AD), and thus x=2, more units of the ligand B will be conjugated to the carrier.

Such conjugation will be able to occur with suitable derivatizations and/or activation of reactive groups of the spacer S and/or the ligand B and/or the carrier, according to methods known to those skilled in the art.

As described above, the conjugates of the invention find use as a medicament.

Particularly, they can be used for the treatment and prevention of cancer.

By the term "treatment" is meant the possibility to administer a pharmacologically efficient amount to a patient having cancer in order to obtain the recovery or an improvement of the patient's conditions.

On the other hand, it is also possible to use the conjugates of the invention for the "prevention" of diseases, in order to prevent the development of a tumor in a patient, especially if susceptible by hereditary, genetic or environmental factors.

In the scope of the present invention, by "primary prevention" is meant the preventive treatment on an healthy subject, while by "secondary prevention" is meant the treatment on an subject who is or has been ill, in order to prevent a new recurrence of the disease.

The tumoral forms on which the compounds of the invention are active preferably comprise breast, colon, prostate, ovary, endometrium, brain, lung, kidney, nose, throat cancer, malignant epithelial tumors, sarcomas, lymphomas, such as Burkitt's lymphoma, Hodgkin's disease, mesotheliomas, melanomas, leukemias and mielomas.

In another aspect of the invention, the described compounds have anti-inflammatory activity, hence they can be used for the treatment and prevention of the diseases characterized or caused by inflammation, such as, for example, rheumatoid arthritis, ulcerative colitis, Crohn's disease, psoriasis, osteomyelitis, multiple sclerosis, atherosclerosis, lung fibrosis, sarcoidosis, systemic sclerosis, transplant rejection and chronic inflammations.

In an additional aspect, the compounds of the invention showed that they have considerable antioxidant properties, hence they are able to reduce the production of free radicals. Such property is particularly useful in those subjects, such as the elderly, smokers, subjects with ongoing diseases or recovering from diseases, in which the generation of radical species is considerably increased. Therefore, the conjugates of the invention can be usefully used for the treatment, cure, and prevention of chronic diseases, such as ischemic disease or inflammatory thrombotic disease.

In another aspect, the conjugates described have a considerable platelet antiaggregating activity.

In a further aspect, the conjugates of the invention can be used for the treatment, prevention and cure of metabolic syndrome, obesity, diabetes, to reduce the body weight and the build-up of fat and of macrophages infiltrated in the adipose tissue, by virtue of the better glucose tolerance that is induced.

According to another aspect, the conjugates of the invention can be used for the treatment and primary and secondary prevention (with the meanings defined above) of cardiovascular diseases.

In a further object, the compounds described in the present patent application are used as food supplements.

For the objects of the present patent application, the use of the compounds described is for both human use and veterinary use, and it preferably is for human use.

One or more of the conjugated compounds of the invention, in a suitable efficient dose, can be formulated in suitable pharmaceutical preparations and, preferably, such compounds are formulated for the oral or parenteral administration.

Said pharmaceutical preparations can, in a particular aspect of the invention, also comprise one or more pharmaceutical active ingredients.

Furthermore, capsules, tablets, lozenges, pills and other similar pharmacological formulations can be made, both for an immediate and a prolonged release, by virtue of the use of suitable excipients.

Instead, for the parenteral administration, it is meant that the compounds of the present invention can be administered via an intradermal, subcutaneous, intramuscular, intraperitoneal, intravenous or intrathecal route.

To such aim, for example, a suitable injectable aqueous solution can be prepared, such as a 5% glucose isotonic solution; alternatively, other pharmaceutically acceptable liquid vehicles can be used.

In a particular aspect, a lyophilized product can be prepared, which can be reconstituted upon use by a suitable vehicle.

One or more of the conjugates of the invention can be further suitably formulated to obtain a product to be used as a food and nutraceutic supplement for one or more of the preventive or therapeutic objects indicated above, by virtue of the ability to release I3C and folic acid, compounds useful for the protection of the human and animal health.

### EXAMPLE 1

### 1a - functionalization of mPEG-OH

The derivatization reaction was carried out starting from monomethoxy-poly(ethylene glycol) linear mPEG-OH with 5 and 20 kDa molecular weight. In the reaction with mPEG_{20,000}-OH, 2.5 g (0.125 mmoles, PM 20 Da) of polymer are dried by distillation of water in the presence of about 40 ml toluene, put to distillate until evaporating about 30-32 ml solvent. 250 µl (0.25 mmoles) of a 1 M solution of potassium tert-butylate in tert-butanol is added under continuous stirring. Refluxing during 1 hour at 50° C and 37 µl (0.25 mmoles, PM 195.06 Da, d = 1.321 g/ml) of tert-butylbromoacetate is added. Reacted during 1 hour at 50° C at reflux and 18 hours at room temperature; filtering on a gooch with a porosity 4 with celite® to remove the Formed KBr, adding first in the reaction flask some mls of CH₂Cl₂ to facilitate the filtration. It is reduced to a small volume by the rotary evaporator, 15 ml of a mixture of TFA (45.4%), CH₂Cl₂ (54.5%) and water (0.1%) is added under stirring and reacted during 3 hours. TFA is removed by rotary evaporator, taking the residue with CH₂Cl₂ for 6-7 times, to a full disappearance of TFA, and it is precipitated dropwise in about 250 ml ethanol; a precipitate is obtained, which is composed of mPEG-COOH, which is let to rest at -20° C during 4-5 hours. Then, filter on gooch with porosity 3 and put in the drier. The same procedure is followed for mPEG₅₀₀₀-OH.

### 1b - derivatization of mPEG_{20,000}-COOH

The active ester derivatization is obtained by reaction with N-hydroxysuccinimide (NHS), in the presence of N,N'-dicyclohexylcarbodiimide (DCC). 800 mg (0.04 mmoles, PM 20058 Da) mPEG_{20,000}-COOH is dissolved in 5-6 ml dry CH₂Cl₂ under stirring and added with 9.21 mg (0.08 mmoles, PM 115.09 Da) N-hydroxysuccinimide (NHS). Once dissolved, 16.51 mg (0.08 mmoles, PM 206,33 Da) N,N'-dicyclohexylcarbodiimide (DCC) is added, and reacted during 18 hours. Filtering on gooch with a porosity 4 to remove the formed dicyclohexylurea and the solution is added dropwise to about 250 ml ethyl ether and put to precipitate at -20° C for 4-5 hours. The formed mPEG-NHS precipitate is filtered on gooch and put in a drier. The mPEG₅₀₀₀-COOH and mPEG₂₂₀₀₀₀-COOH polymers are activated to succinimide ester with the same process.

### 1c - synthesis and purification of the mPEG-I3C conjugates

The mPEG-I3C conjugates, with a different molecular weight and a different structure, are obtained by ester bond between the activated carboxyl group of mPEG and the hydroxyl group of I3C.

The obtained products are characterized by UV-Vis spectrophotometry, ¹H-NMR spectroscopy in DMSO-d6, reverse-phase high-performance liquid chromatography (RP-HPLC).

### Id - synthesis of the folate-PEG-COOH conjugates

The folate-NHS derivative is prepared by esterification of carboxyl in γ of folic acid with N-hydroxysuccinimide (NHS) in anidrous dimethylsulfoxide, in the presence of N,N'-dicyclohexylcarbodiimide (DCC) and triethylamine.

To 500 mg (1.133 mmoles, PM 441.40 Da) of folic acid, dissolved in 10 ml dry DMSO, 240 µl (1.699 mmoles, PM 101.19 Da, d=0.726 g/ml) triethylamine is added, then 260.79 mg (2.266 mmoles, PM 115.09 Da) NHS and 467.54 mg (2.266 mmoles, PM 206.33 Da) DCC, and reacted under stirring away from light overnight. Filtering on gooch with porosity 4 to remove the formed dicyclohexylurea, and the solution is added dropwise to 200 ml ethyl ether and put to precipitate at -20° C for 4 hours. The folate-NHS precipitate is filtered on gooch with porosity 3, washed several times with ethyl ether to remove the residual DMSO, and put in the drier.

The folate-PEG-COOH product is obtained by the formation of an amide bond between the carboxyl group in γ of folic acid, activated to succinimide ester, and the amine group of heterobifunctional PEG. 359.7 mg (0.668 mmoles, PM 538.47 Da) folate-NHS, dissolved in 6 ml dry DMSO, are added dropwise to 800 mg (0.167 mmoles, PM 4800 Da) of NH₂-PEG-COOH, previously solubilized in 6 ml dry DMSO. The reaction pH is adjusted to 9, and it is reacted overnight at room temperature and under stirring. To the reaction mixture 5 ml water is added, the pH is brought to a value of about 7.5, then 5 ml DMF and 2-3 ml saturated NaCl solution are added, to facilitate the phase separation, and the folate-PEG-COOH product is purified from excess folic acid by extracting from the aqueous step with CHCl₃ (8 x 80 ml). The harvested organic step is dried with Na₂SO₄, filtered on gooch to remove the salt, and added dropwise to 200 ml ethyl ether under stirring. After 4 hours at -20° C, the folate-PEG-COOH precipitate is collected by filtration on gooch with porosity 4 and put in the drier.

### 1e - Synthesis and purification of folate-PEG-I3C

The folate-PEG-I3C conjugate is obtained by formation of an ester bond between the activated carboxyl group of PEG and the hydroxyl group of I3C, as set forth above.

### EXAMPLE 2

### Synthesis of the folate-PEG₄₈₀₀- (I3C)₂ conjugates

### 2a - synthesis and purification of NH₂-PEG-AD-(COOH)₂ and NH₂-PEG-βGlu-(COOH)₂

For the conjugating reaction of L-2-aminoadipic (AD) or β-glutamic (β-Glu) acid, a heterobifunctional PEG, Boc-PEG₄₈₀₀-NHS, is used, having the protected amine group at an end and the activated carboxyl group at the other end. The conjugation occurs by formation of an amide bond between the activated carboxyl group of PEG and the amine group of the amino acid.

100.56 mg (0.624 mmoles, PM 161.16 Da) of L-2-aminoadipic acid is dissolved in about 10 ml H₂O/ACN (3:2 v/v), and 87.21 µl (0.624 mmoles, PM 101.19 Da, d=0.726 g/ml) of triethylamine (TEA) is added, so that the reaction pH is 8-9. Then 1 g (0.208 mmoles, PM 4800 Da) of Boc-PEG-NHS is added and reacted under stirring for 3 hours, controlling that the pH is kept at a value of about 8. Next, the reaction is acidified to pH 4.0 with HCl 1 N, the acetonitrile is removed by rotary evaporator, and the Boc-PEG-AD-(COOH)₂ product is purified from the aminoadipic acid excess by extractions with CHCl₃ (8 x 80 ml). The collected organic step is dried with Na₂SO₄, filtered on gooch with porosity 3 to remove the salt, concentrated to a small volume in a rotary evaporator and dropped in about 300 ml ethyl ether under strong stirring. After 4 hours at -20° C, the Boc-PEG-AD-(COOH)₂ precipitate is collected by filtration on gooch with a porosity 4 and put in the drier.

The same process is used in the synthesis of Boc-PEG-βGlu-(COOH)₂. The amino acid amount bound to the PEG chain is determined by titration of the carboxyl groups with 0.01 N and by 1H-NMR spectroscopic characterization. Subsequently, the protecting group Boc is removed by acid hydrolysis with TFA.

### 2b - activation of carboxyl groups of folate-PEG-AD-(COOH)₂ and folate-PEG-βGlu-(COOH)₂ with DCC and NHS

The carboxyl groups of the folate-PEG-AD-(COOH)₂ derivative and folate-PEG-βGlu-(COOH)₂ are activated to succinimide ester with NHS and DCC, as set forth above.

### 2c - synthesis and purification of folate-PEG-AD-(I3C)₂ and folate-PEG-βGlu-(I3C)₂

The conjugation of I3C to the polymer chain occurs by formation of an ester bond between the activated carboxyl groups of the PEG-linked amino acid and the hydroxyl group of I3C, with a process similar to the one followed for the synthesis of folate-PEG-I3C, set forth above.

### EXAMPLE 3

### Optimized I3CPEG₆₀₀I3C synthesis

### 3a - NHS-OOC-PEG₆₀₀-COO-NHS synthesis

PEG₆₀₀ (1.200 g, 2 mmoles) was dissolved in 30 ml toluene and dehydrated by Dean Stark distillation. For the remaining solution PEG (5 ml), anidrous CH₂Cl₂ (70 mL), N-hydroxysuccinimide (NHS) (552.432 mg, 4.8 mmoles) and N,N'-dicyclohexylcarbodiimide (DCC) (990.384 mg, 4.8 mmoles) were added. The mixture was stirred during 18 hours at room temperature. After the filtration of N,N'-dicyclohexylurea, the solvent was removed *in vacuum* to give the product (yield: 1.16 g, 73%).

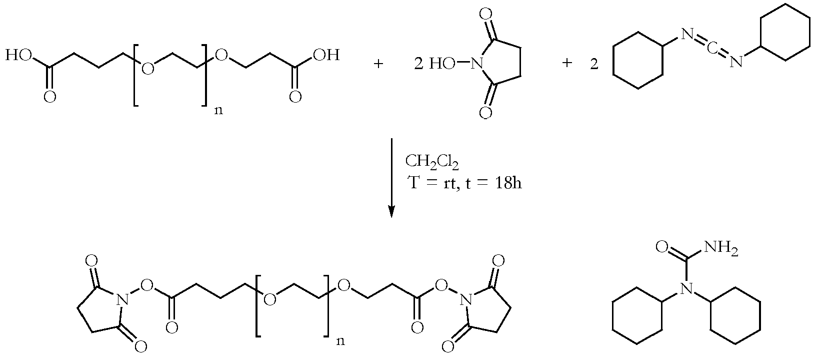

### 3b - synthesis of indole-3-carbinol-PEG600-indole-3-carbinol

Indole-3-carbinol (706.464 mg, 4.8 mmoles) is dissolved in 70 mL dry CH₂Cl₂: to the solution of dry Et₃N (667.2 mL, 4.8 mmoles) and NHS-OOC-PEG₆₀₀-COO-NHS (1 g, 2 mmol) were added and the mixture was stirred during 72 hours at room temperature. The reaction mixture was concentrated *in vacuum.*

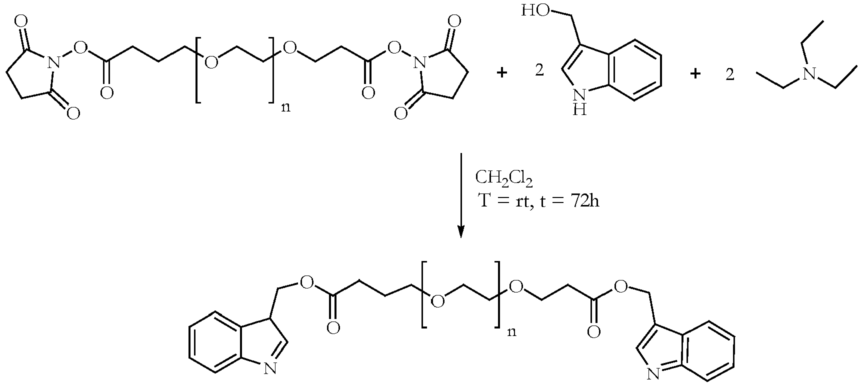

### EXAMPLE 4

### Optimized synthesis of PEG₅₅₀I3C

### 4a- synthesis of PEG₅₅₀-O-CH₂-COOH

PEG₅₅₀ (1.106 mg, 2 mmoles) was added to 30 ml toluene and dehydrated by Dean Stark distillation. For the remaining solution of PEG (5 mL), tert-butanol (10 mL) and potassium tert-butoxide (224.42 mg, 2 mmoles) were added and the solution was left at 50° C during 1 hour, under stirring. Tert-butyl bromoacetate (295.2 mL, 2 mmol) was added and the mixture heated to reflux for 1 hour, reacted during 18 hours at room temperature. KBr was removed by filtration on Celite and the solvent evaporated in vacuum to give mPEG-O-CH₂-COOtBu (yield: 1.33 g, 98%). The ester was hydrolyzed by treatment with 15 ml TFA, 20 mL CH₂Cl₂ and 3 ml H₂O for 3 h. The reaction mixture was concentrated *in vacuum* and TFA was removed under reduced pressure to give mPEG₅₅₀-O-CH₂-COOH (yield: 1.12 g, 92%).

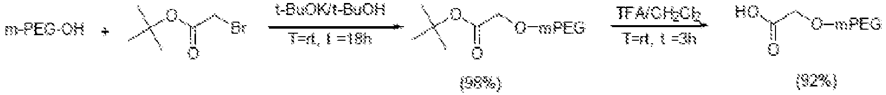

### 4b - synthesis of PEG₅₅₀-O-CH₂-COO-NHS

PEG₅₅₀-(OR-CH₂-COOH)₂ (0.900 g, 1.48 mmol) was dissolved in 75 ml dry CH₂Cl₂. For the solution of N-hydroxysuccinimide (NHS) (204.4 mg, 1.77 mmol) and N,N'-dicyclohexylcarbodiimide (DCC) (366.44 mg, 1.77 mmol) were added. The mixture was stirred for 5 hours at room temperature. After filtration of N,N'-dicyclohexylurea, the solution was added dropwise in 250 ml ethyl ether to precipitate NHS in excess, followed by centrifugation and filtration. The solvent was removed to give PEG₅₅₀-O-CH₂-COO-NHS (yield: 0.987 g, 94.6%).

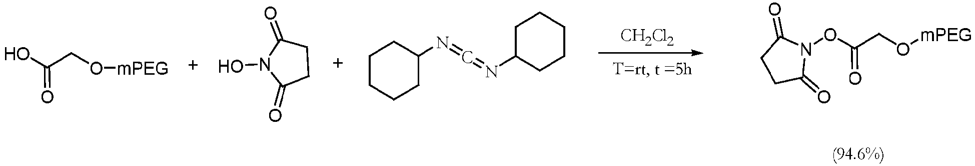

### 3c - synthesis of PEG₅₅₀-indole-3-carbinol

Indole-3-carbinol (273.75 mg, 1.86 mmol) is dissolved in 50 mL dry CH₂Cl₂; to the solution, Et₃N (258.53 ml, 1.86 mmoles) and PEG₅₅₀-O-CH₂COO-NHS (0.987 g, 1.4 mmoles) were added. The mixture was stirred during 72 hours at room temperature. The solvent and Et₃N were removed *in vacuum.*

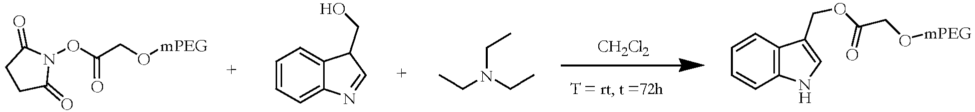

### Studies of biologic activity on F-PEG-I3C conjugates

### EXAMPLE 5

### Cytotoxic activity and targeting ability of the folate conjugates

The studies of cytotoxic activity were carried out on three human cell lines: HT-29 (human colon adenocarcinoma), MCF-7 (human breast adenocarcinoma), and KB-3-1 (human oropharyngeal carcinoma), which cells are characterized by an overexpression of folic acid (FR+) receptors [17-18]. I3C, conjugates free from folic acid (PEG-I3C), and the folate conjugates F-PEG-I3C, F-PEG-AD-(I3C)₂, and F-PEG-βGlu-(I3C)₂ were tested. The data, expressed by IC50, i.e., the concentration (µM) that inhibits the 50% cell growth compared to control cultures, are set forth in Table 1.

**Table 1. IC50 values of I3C and conjugates**

| **COMPOUND** | **HT-29 IC50 (µM)** | **MCF-7 IC50 (µM)** | **KB-3-1 IC50 (µM)** |
|---|---|---|---|
| **I3C** | 45.1±1.7 | 30.0±0.7 | 93.0±2.1 |
| **PEG-I3C** | 62.5±2.1 | 54.1±1.1 | 9 9.1±2.0 |
| **F-PEG-I3C** | 51.3±1.5 | 4 8.5±0.9 | 62.2±1.8 |
| **F-PEG-AD-(I3C)₂** | 51.8±1.1 | 39.7±1.0 | 49.5±1.4 |
| **F-PEG-βGlu-(I3C)₂** | 52.3±1.2 | 41.2±1.8 | 53.3±1.3 |

| | | | |
|---|---|---|---|
| *n=4* number of tests repeated for statistical analysis and calculation of the standard deviation. | | | |

As it can be seen from the IC50 values set forth, in all the three cell lines, the activity of free I3C is comparable to all the conjugates considered. On the other hand, the cytotoxic activity of the F-PEG-I3C conjugates increases when passing from FR⁻ cells to FR⁺ cells, thereby indicating a real targeting effect of the folic acid molecule. Therefore, the conjugates turn out to be strong inhibitors of FR⁺ cells, and such activity is related to a higher selectivity. Next, by comparing the different conjugates one to the other, it is noticed that F-PEG-AD-(I3C)₂ and F-PEG-βGlu-(I3C)₂ have comparable activities, this meaning that the different amino acid spacer used does not affect the cytotoxic activity. Finally, the difference in the activity of these derivatives is very significant when compared to the folate-PEG-I3C conjugate, with only one bound molecule of drug, especially when considering the IC50 values expressed as the concentration of the conjugate.

### EXAMPLE 6

### Anti-inflammatory activity

I3C, conjugate derivatives free from folic acid (PEG-I3C), and folate conjugates F-PEG-I3C, F-PEG-AD-(I3C)₂, and F-PEG-βGlu-(I3C)₂ were tested for their anti-inflammatory activity against the inducible nitric oxide synthetase (iNos) activity in J774A.1 macrophage cells stimulated by LPS and assessing the production of nitrites and the release of NO.

As set forth in Table 2, the activity of the conjugates is comparable to, or a little higher than the one of free I3C. The PEG-I3C conjugates, free from folic acid, show a lower activity, thus indicating a real targeting effect of the folic acid molecule on the activated macrophage cells. Also in this case, F-PEG-AD-(I3C)₂ and F-PEG-βGlu-(I3C)₂ have a comparable activity, pointing out that the different amino acid spacer used is not able to affect the activity. The data are expressed as the inhibition percentage ±s.e.m. vs the production of nitrites during 24 h by J774A.1 macrophage cells treated with LPS alone.

**Table 2. Effect of I3C and the conjugates on the production of nitrites and the release of NO in J774A.1 macrophage cells stimulated with LPS of E. coli.**

| **COMPOUND** | **Nitrites release inhibition (% vs LPS)** | | |
|---|---|---|---|
| | 100 µg/mL | 10 µg/mL | 1 µg/mL |
| **I3C** | 70.1±1.2*** | 20.0±0.9** | 10.0±1.1 |
| **F-PEG-AD-(I3C)₂** | 73.8±3.1*** | 22.7±1.8** | 9.5±1.0 |
| **F-PEG-βGlu-(I3C)₂** | 72.3±2.2*** | 21.2±1.3** | 12.3±1.1 |
| **PEG₅₅₀-I3C** | 72.5±2.4*** | 34.1±1.2** | 12.5±0.8 |
| **I3C-PEG₆₀₀-I3C** | 84.3±3.8*** | 45.7±1.7** | 32.2±1.4** |

| | | | |
|---|---|---|---|
| *n*=5 number of tests repeated for statistical analysis and calculation of the standard deviation; *** and ** represent P<0.001 and P<0.01 vs treated LPS macrophages. | | | |

### EXAMPLE 7

### Platelet antiaggregating activity

The platelet anti-aggregating effect induced by a series of mediators was tested on platelet-enriched plasma samples. I3C and the folate conjugate F-PEG-I3C showed to inhibit in a strong and dose-dependent manner the collagen-induced platelet aggregation. When tested at concentrations of 3, 6, 12, 25 µM, they produced a platelet aggregation inhibition of 70%, 73%, 78% and 79%, and 71%, 75%, 77%, 80%, respectively.

### EXAMPLE 8

### Antioxidant activity

The cationic free radical ABTS+° discoloration assay assesses the scavenging ability of an antioxidant against a preformed cationic radical (ABTS⁺°) compared to Trolox, a hydrosoluble analogue of Vitamin E. TEAC is defined as the concentration of Trolox having the same antioxidant ability of the tested compound at a 1 mM concentration. The folate conjugates F-PEG-I3C showed an antioxidant activity that is higher than that of I3C and a number of polyphenolic compounds, confirming the higher stabilization of I3C. F-PEG-AD-(I3C)₂ and F-PEG-βGlu-(I3C)₂ have a superior and comparable activity, pointing out that the different amino acid spacer used is not capable of affecting this ability.

| **Class** | **Compound** | **ABTS assay (TEAC)** |
|---|---|---|
| **I3C** | I3C | 3.8 ±0.12 |
| | PEG₅₅₀-I3C | 4.2 ±0.26 |
| | I3C-PEG₆₀₀-I3C | 5.8 ±0.41 |
| | F-PEG-I3C | 4.5 ±0.21 |
| | F-PEG-AD-(I3C)₂ | 5.3 ±0.21 |
| | F-PEG-βGlu-(I3C)₂ | 5.2 ±0.18 |
| **PPG** | Verbascoside | 5.3 ±0.22 |
| | | |
| **Flavonoids** | Rutin | 2.1 ±0.09 |
| | Hesperidin | 0.5 ±0.09 |
| | Naringin | 0.5 ±0.01 |
| | Naringenin | 1.4 ±0.03 |
| | Apigenin | 0.2 ±0.01 |
| | Epicatechin | 4.8 ±0.08 |
| | Luteolin | 2.8 ±0.09 |
| | | |
| **Cinnamic acids** | Caffeic acid | 5.8 ±0.12 |
| | Chlorogenic acid | 4.3 ±0.13 |
| | Ferulic acid | 3.6 ±0.11 |
| | 3,4-Dihydroxybenzoic acid | 4.6 ±0.18 |
| | | |
| **Vitamins** | Tocopherol | 2.1 ±0.07 |

From the description set forth above, the various advantages provided by the present invention are apparent.

In fact, the conjugates described overcome some limitations of the indole-3-carbinol, relating its low molecular weight and its instability in water and in an acidic environment, as the one represented by the stomach.

In the conjugates of the invention, I3C is covalently linked to a polymer acting as a carrier, and such conjugation allows improving its pharmacokinetic profile.

Consequently, the solubility is advantageously increased, and the enzymatic and chemical degradation is considerably reduced or completely avoided.

Furthermore, the compounds provided by the present invention are provided with a high selectivity, by virtue of the use of targeting molecules.

Such targeting molecule is recognized by specific receptors that are overexpressed in some tissues and cells, such as, for example, particular tumor and inflammatory cells.

Consequently, the toxic and cytotoxic effect on the tissues not affected by a tumor or inflammatory disease is advantageously prevented.

The antioxidant properties of the compounds described above lead to the use thereof as food supplements.

In fact, taking exogenous antioxidants concurs in restoring the balance between the free radical production and the consumption of endogenous antioxidants, in addition to preventing the unbalance that causes the so-called "oxidative cell stress", which is related to both the physiological ageing process and environmental insults.

## Claims

1. Compound of the general formula:
**Bₓ-(S)_{y}-L-(S')_{z}-Dₙ** **(I)**
wherein:
**B** is folic acid (_{F});
**x** is 0, 1, or 2;
**S** is lysine, or it is a bifunctional carrier, such as L-2-aminoadipic acid (AD);
**y** is 0 or 1 or 2;
**L** is a carrier selected from polyethylene glycol or a homobifunctional or heterobifunctional derivative thereof;
**S'** is a bifunctional carrier selected from L-2-aminoadipic acid (AD) and β-glutamic acid (β-Glu);
**z** is 0 or 1 or 2;
**D** is an active ingredient selected from indole-3-carbinol (I3C) or a derivative thereof which is a N-acyl derivative, comprising a straight or branched C₁-C₆ carbonyl chain and preferably C₁; N-alkoxy comprising a straight or branched C₁-C₆ carbonyl chain and preferably represented by: N-methoxy, N-ethoxy, N-propoxy, N-butoxy; N-aryl or N-heteroaryl derivatives comprising one or more heteroatoms selected from nitrogen, oxygen and sulphur, N-benzyl derivatives; N-alkyl derivatives comprising a straight or branched C₁-C₆ carbonyl chain, optionally comprising one or more heteroatoms selected from nitrogen, oxygen and sulphur; N-alkenyl or N-alkinyl derivatives comprising a straight or branched C₂-C₆ carbonyl chain comprising one or more insaturations and optionally further comprising one or more heteroatoms selected from nitrogen, oxygen and sulphur ;
**n** is 1 or 2 or 3 or 4.

2. The compound according to any of the preceding claims, wherein the carrier is H₂N-(PEG)ₙ-COOH or HOOC-(PEG)ₙ-COOH.

3. The compound according to any of the preceding claims, wherein the carrier *is* monomethoxy-polyethylene glycol.

4. The compound according to any of the preceding claims, wherein the carrier has a molecular weight ranging between 100 and 200,000 (g/mol), and preferably between 4,800 and 40,000 (g/mol).

5. The compound according to the preceding claim, wherein the carrier has a molecular weight of 550, 600, 4,800, or 20,000.

6. The compound according to any of the preceding claims, selected from:
**PEG-I3C**
**I3C-PEG-I3C**
**PEG-AD-(I3C)₂**
**PEG-βGLU-(I3C)₂**
**B-Lys-PEG-I3C**
**B-Lys-PEG-AD-(I3C)₂**
**B-Lys-PEG-βGLU-(I3C)₂**
**F-PEG-I3C**
**F-PEG-AD-(I3C)₂**
**(I3C)₂-AD-PEG-AD-(I3C)₂**
**F-PEG-βGLU-(I3C)₂**
**F-Lys-PEG-I3C**
**F-Lys-PEG-AD-(I3C)₂**
**F-Lys-PEG-βGLU-(I3C)₂**
**F₂-AD-PEG-I3C**
**F₂-AD-PEG-βGLU-(I3C)₂**
**F₂-AD-PEG-AD-(I3C)₂**
wherein F is folic acid; Lys is lysine; PEG is polyethylene glycol or a derivative thereof, such as monomethoxy polyethylene glycol (mPEG), homobifunctional or heterobifunctional; wherein AD is L-2-aminoadipic acid and β-Glu is β-glutamic acid and wherein I3C is the indole-3-carbinol (I3C) or derivative thereof.

7. A compound according to any of the claims 1 to 7 for medical use.

8. A compound according to any of the claims 1 to 7, for use in the treatment or prevention of a tumor.

9. The compound according to the preceding claim, wherein said tumor is a breast, colon, prostate, ovary, endometrium, brain, lung, kidney, nose, throat tumor, malignant epithelial tumors, sarcomas, lymphomas, such as Burkitt's lymphoma, Hodgkin's disease, mesotheliomas, melanomas, leukemias, and mielomas.

10. The compound according to claim 7, for use in the prevention and treatment of inflammatory diseases, cardiovascular diseases, chronic diseases, metabolic syndrome, obesity, diabetes, to decrease the body weight and the build-up of fat and macrophages infiltrated in the adipose tissue, for use as a platelet anti-aggregating agent, for use as an antioxidant.

11. A pharmaceutical or nutraceutical preparation comprising one or more of the compounds according to any of the claims 1 to 6.

12. The pharmaceutical or nutraceutical preparation according to the preceding claim, for oral or parenteral administration.

13. The pharmaceutical or nutraceutical preparation according to claim 11 or 12, comprising one or more pharmaceutical active ingredients.

14. The compound according to any of the claims 1 to 6, for use as a food and nutraceutical supplement capable of releasing I3C and folic acid that are useful for the human and animal health.

## Patentansprüche

1. Verbindung der allgemeinen Formel:
**Bₓ-(S)_{y}-L-(S')_{z}-Dₙ** **(I)**
wobei:
B Folsäure (F) ist;
x 0, 1 oder 2 ist;
S Lysin ist oder es ein bifunktioneller Träger wie L-2-Aminoadipinsäure (AD) ist;
y 0 oder 1 oder 2 ist;
L ein Träger ist, ausgewählt aus Polyethylenglykol oder einem homobifunktionellen oder heterobifunktionellen Derivat davon;
S' ein bifunktioneller Träger ist, ausgewählt aus L-2-Aminoadipinsäure (AD) und β-Glutaminsäure (β-Glu);
z 0 oder 1 oder 2 ist;
D ein Wirkstoff ist, ausgewählt aus Indol-3-Carbinol (I3C) oder einem Derivat davon, das ein N-Acetyl Derivat ist, umfassend eine gerade oder verzweigte C₁-C₆-Carbonylkette und vorzugsweise C₁; N-Alkoxy, umfassend eine gerade oder verzweigte C₁-C₆-Carbonylkette und vorzugsweise dargestellt ist durch: N-Methoxy, N-Ethoxy, N-Propoxy, N-Butoxy; N-Aryl oder N-Heteroaryl Derivate, umfassend ein oder mehrere Heteroatome, ausgewählt aus Stickstoff, Sauerstoff und Schwefel, N-Benzyl Derivate; N-Alkyl Derivate, umfassend eine gerade oder verzweigte C₁-C₆-Carbonylkette, gegebenenfalls umfassend ein oder mehrere Heteroatome, ausgewählt aus Stickstoff, Sauerstoff und Schwefel; N-Alkenyl oder N-Alkinyl Derivate, umfassend eine gerade oder verzweigte C₂-C₆-Carbonylkette, umfassend eine oder mehrere Ungesättigtheiten und gegebenenfalls ferner umfassend ein oder mehrere Heteroatome ausgewählt aus Stickstoff, Sauerstoff und Schwefel;
n 1 oder 2 oder 3 oder 4 ist.

2. Verbindung nach einem der vorhergehenden Ansprüche, wobei der Träger H₂N-(PEG)ₙ-COOH oder HOOC-(PEG)ₙ-COOH ist.

3. Verbindung nach einem der vorhergehenden Ansprüche, wobei der Träger Monomethoxypolyethylenglykol ist.

4. Verbindung nach einem der vorhergehenden Ansprüche, wobei der Träger ein Molekulargewicht im Bereich zwischen 100 und 200.000 (g/mol) und vorzugsweise zwischen 4.800 und 40.000 (g/mol) aufweist.

5. Verbindung nach dem vorhergehenden Anspruch, wobei der Träger ein Molekulargewicht von 550, 600, 4.800 oder 20.000 aufweist.

6. Verbindung nach einem der vorhergehenden Ansprüche, ausgewählt aus:
**PEG-I3C**
**I3C-PEG-I3C**
**PEG-AD-(I3C)₂**
**PEG-βGLU-(I3C)₂**
**B-Lys-PEG-I3C**
**B-Lys-PEG-AD-(I3C)₂**
**B-Lys-PEG-βGLU-(I3C)₂**
**F-PEG-I3C**
**F-PEG-AD-(I3C)₂**
**(I3C)₂-AD-PEG-AD-(I3C)₂**
**F-PEG-βGLU-(I3C)₂**
**F-Lys-PEG-I3C**
**F-Lys-PEG-AD-(I3C)₂**
**F-Lys-PEG-βGLU-(I3C)₂**
**F₂-AD-PEG-I3C**
**F₂-AD-PEG-βGLU-(I3C)₂**
**F₂-AD-PEG-AD-(I3C)₂**
wobei F Folsäure ist; Lys Lysin ist; PEG Polyethylenglykol oder ein Derivat davon ist, wie Monomethoxypolyethylenglykol (mPEG), homobifunktionell oder heterobifunktionell; wobei AD L-2-Aminoadipinsäure ist und β-Glu β-Glutaminsäure ist und wobei I3C das Indol-3-Carbinol (I3C) oder Derivat davon ist.

7. Verbindung nach einem der Ansprüche 1 bis 7 zur medizinischen Verwendung.

8. Verbindung nach einem der Ansprüche 1 bis 7 zur Verwendung bei der Behandlung oder Vorbeugung eines Tumors.

9. Verbindung nach dem vorhergehenden Anspruch, wobei der Tumor ein Brust-, Dickdarm-, Prostata-, Eierstock-, Gebärmutterschleimhaut-, Gehirn-, Lungen-, Nieren-, Nasen-, Rachen-Tumor, maligne epitheliale Tumore, Sarkomen, Lymphome, wie Burkitt-Lymphom, Morbus Hodgkin, Mesotheliome, Melanome, Leukämien und Myelome ist/sind.

10. Verbindung nach Anspruch 7 zur Verwendung bei der Vorbeugung und Behandlung von entzündlichen Erkrankungen, Herz-Kreislauf-Erkrankungen, chronischen Erkrankungen, metabolischem Syndrom, Fettleibigkeit, Diabetes, um das Körpergewicht und den Aufbau von Fett und Makrophagen, infiltriert in das Fettgewebe, zu verringern, zur Verwendung als Blutplättchen-Antiaggregationsmittel, zur Verwendung als Antioxidans.

11. Pharmazeutische oder nutrazeutische Zubereitung, umfassend eine oder mehrere der Verbindungen nach einem der Ansprüche 1 bis 6.

12. Pharmazeutische oder nutrazeutische Zubereitung nach dem vorhergehenden Anspruch zur oralen oder parenteralen Verabreichung.

13. Pharmazeutische oder nutrazeutische Zubereitung nach Anspruch 11 oder 12, umfassend einen oder mehrere pharmazeutische Wirkstoffe.

14. Verbindung nach einem der Ansprüche 1 bis 6 zur Verwendung als ein Nahrungsmittel und Nahrungsergänzungsmittel, die in der Lage sind I3C und Folsäure freizusetzen, die für die humane und Tiergesundheit nützlich sind.

## Revendications

1. Composé de formule générale :
Bₓ-(S)_{y}-L-(S')_{z}-Dₙ (I)
dans laquelle :
B est l'acide folique (F) ;
x est égal à 0, 1 ou 2 ;
S est une lysine, ou un support bifonctionnel, tel que l'acide L-2-aminoadipique (AD) ;
y est égal à 0 ou 1 ou 2 ;
L est un support choisi parmi le polyéthylène glycol ou un dérivé homobifonctionnel ou hétérobifonctionnel de celui-ci ;
S' est un support bifonctionnel choisi parmi l'acide L-2-aminoadipique (AD) et l'acide β-glutamique (β-Glu) ;
z est égal à 0 ou 1 ou 2 ;
D est un ingrédient actif choisi parmi l'indole-3-carbinol (I3C) ou un dérivé de celui-ci qui est un dérivé N-acyle, comprenant une chaîne carbonyle en C₁-C₆ linéaire ou ramifiée et de préférence en C₁ ; un N-alcoxy comprenant une chaîne carbonyle en C₁-C₆ linéaire ou ramifiée, et représenté de préférence par : N-méthoxy, N-éthoxy, N-propoxy, N-butoxy ; des dérivés N-aryles ou N-hétéroaryles comprenant un ou plusieurs hétéroatomes choisis parmi l'azote, l'oxygène et le soufre, des dérivés N-benzyles ; des dérivés N-alkyles comprenant une chaîne carbonyle en C₁-C₆ linéaire ou ramifiée, comprenant facultativement un ou plusieurs hétéroatomes choisis parmi l'azote, l'oxygène et le soufre ; des dérivés N-alcényles ou N-alcynyles comprenant une chaîne carbonyle en C₂-C₆ linéaire ou ramifiée comprenant une ou plusieurs insaturations et comprenant facultativement en outre un ou plusieurs hétéroatomes choisis parmi l'azote, l'oxygène et le soufre ;
n est égal à 1 ou 2 ou 3 ou 4.

2. Composé selon l'une quelconque des revendications précédentes, dans lequel le support est H₂N-(PEG)ₙ-COOH ou HOOC-(PEG)ₙ-COOH.

3. Composé selon l'une quelconque des revendications précédentes, dans lequel le support est un monométhoxy-polyéthylène glycol.

4. Composé selon l'une quelconque des revendications précédentes, dans lequel le support a un poids moléculaire compris entre 100 et 200 000 (g/mol), et de préférence entre 4 800 et 40 000 (g/mol).

5. Composé selon la revendication précédente, dans lequel le support a un poids moléculaire de 550, 600, 4 800 ou 20 000.

6. Composé selon l'une quelconque des revendications précédentes, choisi parmi :
PEG-I3C
I3C-PEG-I3C
PEG-AD-(I3C)₂
PEG-βGLU-(I3C)₂
B-Lys-PEG-I3C
B-Lys-PEG-AD-(I3C)₂
B-Lys-PEG-βGLU-(I3C)₂
F-PEG-I3C
F-PEG-AD-(I3C)₂
(I3C)₂-AD-PEG-AD-(I3C)₂
F-PEG-βGLU-(I3C)₂
F-Lys-PEG-I3C
F-Lys-PEG-AD-(I3C)₂
F-Lys-PEG-βGLU-(I3C)₂
F₂-AD-PEG-I3C
F₂-AD-PEG-βGLU-(I3C)₂
F₂-AD-PEG-AD-(I3C)₂
dans lequel F est l'acide folique ; Lys est une lysine ; PEG est le polyéthylène glycol ou un dérivé de celui-ci, tel qu'un monométhoxy polyéthylène glycol (mPEG), homobifonctionnel ou hétérobifonctionnel ; dans lequel AD est l'acide L-2-aminoadipique et β-Glu est l'acide β-glutamique et dans lequel I3C est l'indole-3-carbinol (I3C) ou un dérivé de celui-ci.

7. Composé selon l'une quelconque des revendications 1 à 7 pour une utilisation médicale.

8. Composé selon l'une quelconque des revendications 1 à 7, pour une utilisation dans le traitement ou la prévention d'une tumeur.

9. Composé selon la revendication précédente, dans lequel ladite tumeur est une tumeur du sein, du colon, de la prostate, de l'ovaire, de l'endomètre, du cerveau, du poumon, du rein, du nez, de la gorge, des tumeurs épithéliales malignes, des sarcomes, des lymphomes, tels que le lymphome de Burkitt, la maladie de Hodgkin, des mésothéliomes, des mélanomes, des leucémies, et des myélomes.

10. Composé selon la revendication 7, pour une utilisation dans la prévention et le traitement de maladies inflammatoires, de maladies cardiovasculaires, de maladies chroniques, du syndrome métabolique, de l'obésité, du diabète, afin de réduire le poids corporel et l'accumulation de graisse et de macrophages infiltrés dans le tissu adipeux, pour une utilisation comme agent antiagrégant plaquettaire, pour une utilisation comme antioxydant.

11. Préparation pharmaceutique ou nutraceutique comprenant un ou plusieurs des composés selon l'une quelconque des revendications 1 à 6.

12. Préparation pharmaceutique ou nutraceutique selon la revendication précédente, pour une administration orale ou parentérale.

13. Préparation pharmaceutique ou nutraceutique selon la revendication 11 ou 12, comprenant un ou plusieurs ingrédients actifs pharmaceutiques.

14. Composé selon l'une quelconque des revendications 1 à 6, pour une utilisation comme complément alimentaire et nutraceutique capable de libérer de l'I3C et de l'acide folique qui sont utiles pour la santé humaine et animale.
